# EUROPEAN PATENT SPECIFICATION

(11) **EP 0 602 351 B1**
(45) Date of publication and mention of the grant of the patent: **10.03.1999**
(21) Application number: 93117120.1
(22) Date of filing: 22.10.1993
(51) Int. Cl.: A61B 17/70

(54) **A device for repositioning vertebras of the human spine**
Vorrichtung zum Reponieren von Wirbeln des menschlichen Rückgrates
Dispositif de repositionnement de vertèbres de l'épine dorsale humaine

(30) Priority: 16.12.1992 DE 9217171 U
(43) Date of publication of application: 22.06.1994
(73) Proprietor: Howmedica GmbH, D-24232 Schönkirchen (DE)
(72) Inventor: Bertagnoli, Rudolf, Dr. med., D-37073 Göttingen (DE); Harder, Hans Erich, D-24253 Probsteierhagen (DE)
(74) Representative: Patentanwälte Hauck, Graalfs, Wehnert, Döring, Siemons

(56) References cited:
- DE-C- 4 108 918
- US-A- 4 926 849

## Description

The present invention relates to a device for repositioning vertebras of the human spine.

### Background of the Invention

A number of implants and devices are known to remedy injuries and deformations of spines. For this purpose, the vertebras concerned are stabilized with respect to each other by sophisticated operations. Usually so-called pedicel screws are used in pairs which are screwed in a vertebra through the pedicel. Before stabilizing the vertebra it is regularly necessary to reposition one or more of the vertebras concerned. When the spine is bent (kyphosis) it is necessary for example, to outwardly displace one or more vertebras relative to others. For this purpose, known devices are available to the surgeon which cooperate alike with pedicel screws, for example. The known devices, however, need high forces executed by the surgeon.

### Prior Art

DE 41 08 918 C1 discloses a device for repositioning vertebras, in which a cross piece is supported on a rod or a bar through a pair of legs slidably guiding the cross piece, which bar is connected to the vertebras in a known manner. The cross piece slidably guides a single draw bar which is provided with a hook-shaped gripper. The gripper has a slot for engaging the head of pedicel screw screwed in a vertebra from below. As the pedicel screw cannot be centrally screwed in the vertebra, but from the side, a rotation is exerted to the vertebra which may adversally affect a proper repositioning.

### Summary of the Invention

The present invention is based on the object, to provide a device for repositioning vertebras of the human spine which not only facilitates effective repositioning, needing little force executed by the surgeon, however, is also to allow rotation of the spine or the vertebra, respectively.

This problem is solved by claims 1 and 2.

The device according to claim 1 comprises a horizontal yoke-like element which is connected to a pair of horizontally distant legs. The horizontal element extends above the patient lying on the stomach on a table and carries lifting means. Two tie elements are connected to the lifting means with the lower end of the tie elements including gripping means for a holding element to be connected to a vertebra.

According to the alternative embodiment of claim 2, the lower end of the legs of the yoke element extending lengthwise of the lying patient are provided with base portions, of which one includes means for being mounted in the sacrum by means of a sacrum screw extending through a hole of the base portion. The front leg has a base portion which is ring-shaped to be placed on the shoulder of the patient.

For transferring tie forces it is useful to provide a pair of adjacent tie elements for a vertebra each. Not only uniform tie forces or compressive forces may be exerted to the vertebra thereby, but further a desired torque for rotating the vertebra about a desired angle may be exerted as well by means of both the tie elements. Subsequently the tie elements may be uniformly loaded again.

The invention is based on the observation that a vertebra when being lifted may be displaced relative to an adjacent vertebra. As a larger portion of the patient's weight is carried by the lifted vertebra, this very vertebra is subjected to a substantial repositioning force in this manner which may be sufficient to provide the desired adjustment of the vertebra. When the force should be still increased, the surgeon may press the patient against the table or may fix the patient to the table by suitable means.

As far as the statement is concerned that the tie elements are adjustable in height with respect to the yoke element, this means as well to adjust the horizontal portion relative to the legs. The adjustment of the tie elements, preferably sidewards as well, allows to adjust the distance of the tie elements with respect to the vertebra which is connected to the holding means, preferably pedicel screws. As said before, the legs must be suitably supported on the table or the patient. In the invention the support includes a pair of legs standing along the length sides of a table, the upper ends of the legs being interconnected by the yoke element. The legs may be removably mounted to the table to provide for an adjustment with respect to the vertebra concerned. Preferably, the legs are horizontally slidably arranged to facilitate the necessary adjustment. For example, rails or the like may be provided along the edges of the table, on which rails the legs slide to be secured in a desired position. Additionally or alternatively, the yoke element is designed to be horizontally displaced in limits with respect to the legs and to be fixed at desired positions. Furthermore, the yoke element may be designed to carry distant pairs of tie elements to be adjustable in height in order to provide a simultaneous repositioning of a number of vertebras. In this case it is useful to vary the distance between the pairs of tie elements.

In case that the yoke element is not adjustable in height the tie elements may be fixed to the lifting means. The lifting means may include a lifting plate for example, on which the tie elements are mounted, wherein the lifting plate is adjusted in height relative to the yoke element by means of a spindle.

According to a further embodiment of the invention, the legs are pivotally supported and may be secured in their angular positions. Preferably, the horizontal holding portion or, respectively the yoke element is also pivoted relative to the leg so that the tie elements may be arranged substantially vertical. Pivoting the support facilitates the access to the field of operation.

It is useful to suspend the tie elements like a pendulum to facilitate the handling with the pedicel screw and to provide a compensation for an inaccurate adjustment. According to an embodiment of the invention it is useful to provide the tie elements in two parts, of which one is connected to the horizontal yoke element and the second is floatingly suspended on the first part. The first part of the tie elements may be connected to the yoke element, for example to the lifting plate in a relatively stiff fashion, to be adjusted in height by the lifting plate.

Pedicel screws have ring-like heads are known. It is thus possible to shape the lower ends of the tie elements like hooks to engage the ring-like heads. Alternatively, the tie elements may be formed like a fork to engage the head of the pedicel screw from below.

In the alternative embodiment of the invention, wherein the legs are placed on the patient, at least one leg may be pivotally supported on the yoke element. Accordingly, the leg may be adjusted to the length axis of the screw provided to support the leg. The horizontal yoke element interconnecting the legs may be arranged telescopically to fit to the length of the patient.

### Brief Description of the Drawings

In the following the invention is described with reference to the drawings.
- Fig. 1: shows schematically a first embodiment of the invention.
- Fig. 2: shows a side view of a second embodiment of the invention.
- Fig. 3: shows a plane view of the embodiment of Fig. 2.
- Fig. 4: shows schematically a side view of a portion of the support shown in Fig. 1 in the direction of the arrow 4.
- Fig. 5: shows a plane view of a schematically illustrated vertebra including a holding element.

### Detailed Description of the Drawings

It is emphasized that the drawings are of a very schematical nature and not drawn to scale.

A patient 10 whose silhouette is shown in dashed lines is placed with the stomach on an operation table not shown. Lengthside of the table there are mounted rails 12, 14 supporting a plurality of vertical legs 16, 18. The legs 16, 18 engage the rails 12, 14 in a U-shaped fashion so that the legs 16, 18 may be horizontally displaced. By means of clamping screws 20, 22 the legs 16, 18 may be fixed in the desired position. The legs extend through elongate holes of a cross piece plate. The elongate holes 24, 26 extend vertically with respect to the plane of the drawing. The plate 28 is supported on flanges 30, 32 of the legs 16, 18 and the upper portions of the legs 16, 18 are threaded to receive a butterfly nut 34, 36 to secure the plate 28 to the legs 16, 18. When the nut 34, 36 is released, the plate 28 may be horizontally displaced vertical to the plane of the drawing. Central in the plate there is provided an elongate slot 38 through which a pair of tie elements 40, 42 extends. The tie elements comprise an upper part 44, 46 and a lower part 48, 50. The upper parts 44, 46 extending through the slot 38 and through holes (not shown) of a lifting plate 52 are fixed there to by means of nuts 54, 56. By rotating the nuts 54, 56 the parts 44, 46 may be adjusted in height. The lifting plate has a threaded bore for receiving a threaded spindle 58 which lower end is supported on a counter plate 60 of the cross plate 28. The upper end of the spindle 58 carries a knurled head 62. By rotating the spindel 58 the lifting plate 52 may be adjusted in height. Accordingly the height of the tie elements 40, 42 is adjusted.

As one may recognize, the upper parts 44, 46 of the tie elements 40, 42 are ring-like shaped and the lower parts 48, 50 engage with hooks the annular portions to be suspended like a pendulum. The lower parts end in forke-like hook portions 64, 66 for engaging the head 68 or 70 of a pedicel screw 72 or 74 from below. The pedicel screws 72, 74 are screwed in the pedicel of a vertebra of the human spine in a manner not shown.

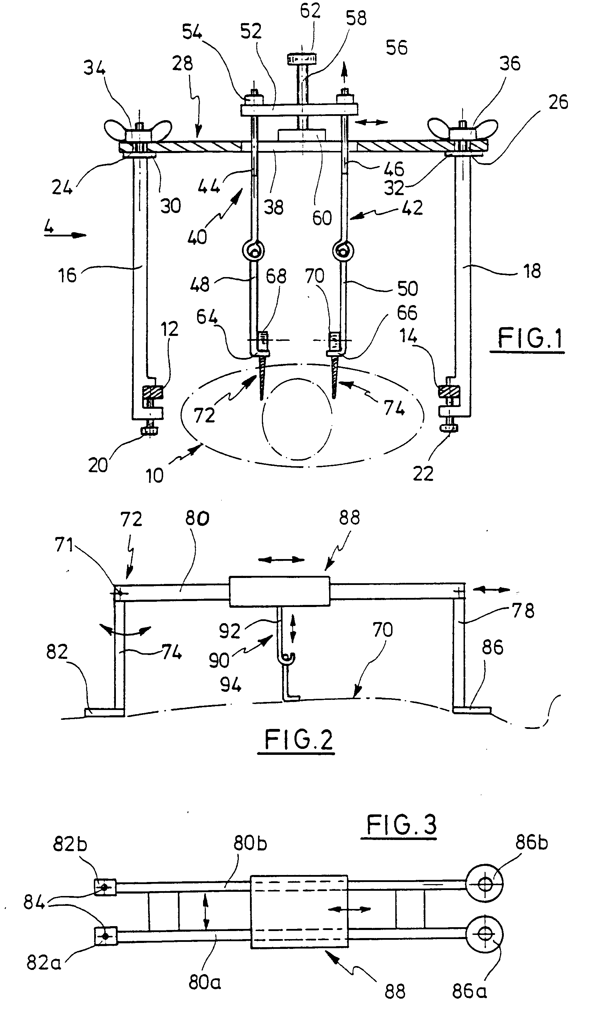

For exerting a tensile force to the tie elements 40, 42 through the spindle 58 and the lifting plate 52, a tensile force may act on the vertebra concerned to adjust the very vertebra relative with respect to the adjacent vertebras. Of course, a plurality of devices shown in Fig. 1 may be provided or, respectively a number of pairwise arranged tie elements may be used to exert a tensile force to a plurality of vertebras in the manner described.

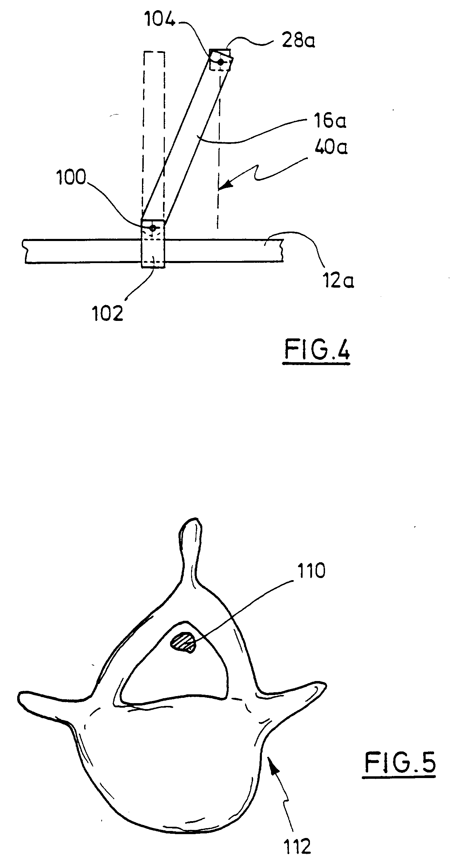

Fig. 4 shows a leg 16a similar to leg 16 of the support shown in Fig. 1, which leg 16a is pivotally arranged at 100 to a portion 102 which is slidably arranged on the rail 12a to be fixed thereto. The leg 16a may be locked in any pivotal position. The means to accomplish this, are not shown. One realizes that the tie or pressure elements 40a shown in dashed lines extend remote from the fixing part of the legs to facilitate the access for the surgeon.

As Fig. 4 further shows, the cross piece 28a is pivotally supported at 104 to the legs. This pivotal adjustment is useful when the tie or pressure elements are not pivotally supported on the cross piece, but are rigidly secured thereto at least with their upper parts. Instead of pivoting the cross piece 28a, an upper pivotal portion for the leg may be provided.

Fig. 2 shows the silhouette 70 of a patient lying with the stomach on an operation table not shown. A support 72 comprises vertical legs 74, 78 having upper ends being connected together through a cross piece 80. The cross piece 80 may be arranged telescopically to vary the distance of the legs 74, 78. As Fig. 3 shows, the device shown in Fig. 2 is doubled, each half designated by adding a and b. The lower end of the leg 74 includes a placing portion 82 having a hole 84 through which a sacrum screw may extend for mounting the leg 74 to the sacrum of the spine. The lower end of the front leg 78 has a ring-shaped placing face 86 through which the leg 78 is placed on the shoulder of the patient.

As indicated at 71, the leg 74 is pivoted relative to the cross piece 80. Thereby the axis of the leg 74 may be aligned to the axis of the screw.

Both cross pieces 80a, 80b support a slide 88 which is horizontally slidable. The slide 88 carries a pair of tie elements one of which is shown in Fig. 2 at 90. Each tie element comprises again an upper part 92 and a lower part 94 which are similarly designed as the tie elements 40, 42 shown in Fig. 1 performing the same function. The tie elements 90 are adjustable in height with respect to the slide 88 in a manner not shown to provide a tensile force to a vertebra in a manner described above.

Fig. 5 shows a wire loop 110 which extends through a hole of a vertebra 112. By means of the wire loop 110 a tensile force may be exerted on the vertebra 112. The wire loop 110 is connected to one or two tie elements of the type shown in figures 1 and 2.

## Claims

1. A device for reposition vertebraes of the human spine, comprising
- a yoke-like elongated element (28) extending horizontally,
- vertical legs (16, 18) attached to the ends of the yoke element (28), said legs being constructed to be supported along opposed longitudinal sides of a table which is adapted to support a patient lying thereon, said legs being displaceable relative to said table,
- lifting means (58, 62) on said yoke element (28),
- two tie elements (40, 42) connected to the lifting means (62, 58, 52) in parallel extending spaced relationship and having gripping means (64, 66) at the free ends thereof adapted to engage holding means (72, 74) attached to a vertebra.

2. A device for repositioning vertebraes of the human spine, comprising
- a yoke-like elongated element (80, 80a, 80b) extending horizontally,
- vertical legs (74, 78) attached to the ends of the yoke element (80, 80a, 80b), said legs (74, 78) having base portions (82, 86, 82a, 82b, 86a, 86b) at the lower ends thereof, one (92, 82a, 82b) of said base portions having means adapted to be attached to the sacrum of a patient through a sacrum screw extending through a hole (84) of said base portion (82, 82a, 82b) and the other (86, 86a, 86b) base portions being ring-shaped in order to be supported by the shoulder portion of a patient,
- slide means (88) constructed to be displaced along said yoke element (80, 80a, 80b),
- two tie elements (90) connected to said slide means (88) in parallel extending spaced relationship, and adapted to be adjustable in height, said tie elements (90) having gripping means at the free ends thereof adapted to engage holding means attached to a vertebra.

3. The device of claim 1 or 2, wherein said holding means is defined by a pedicle screw or a wire pulled through the hole of a vertebra, said holding means cooperating with said tie elements through a hook attached thereto.

4. The device of one of the claims 1 to 3, wherein said legs (16, 18) are releasably attachable to said table.

5. The device of one of the claims 1 to 4, wherein said legs (16, 18) are detachably mounted on said yoke element (28).

6. The device of one of the claims 1 to 5, wherein said legs (16a) are pivotal relative to said yoke element (28) or said table, respectively.

7. The device of claim 6, wherein said yoke element (28a) is pivotal relative to said legs about a longitudinal axis or an axis parallel to the longitudinal axis of said yoke element.

8. The device of one of the claims 1 to 7, wherein said legs (16, 18) are displaceable relative to said yoke element through elongate holes (24, 26) and may be fixedly attached in any position.

9. The device of one of the claims 1 to 8, wherein said tie elements (40, 42, 90) are adjustable in their length.

10. The device of claim 8 or 9, wherein said tie elements (40, 42) are attached to a lifting plate (52) which is adjustable in height by means of a spindle (58) relative to said yoke element (28).

11. The device of one of the claims 1 to 10, wherein said tie elements (40, 42, 90) consist of a pair of parts (44, 48, 46, 50, 92, 94), one part being connected to said lifting means and said second part being floatingly suspended on the first part.

12. The device of claim 11, wherein the lower ends of said tie elements have fork-like hooks for engaging heads (68, 70) of pedicle screws (72, 74) from below or to carry hooks for engagement with a loop-shaped holding wire.

13. The device of one of the claims 2 to 12, wherein at least a leg (74) is pivotal relative to said yoke element (80) but may be fixedly secured in any angular position.

## Patentansprüche

1. Vorrichtung zum Einrichten von Wirbeln der menschlichen Wirbelsäule, mit
- einem länglichen, jochartigen Element (28), das horizontal verläuft,
- senkrechten Beinen (16, 18), die an den Enden des Jochelementes (28) befestigt sind und so aufgebaut sind, daß sie an gegenüberliegenden Längsseiten eines Tisches gehalten sind, der zum Tragen eines darauf liegenden Patienten ausgebildet ist, wobei die Beine gegenüber dem Tisch verschiebbar sind,
- einer Hubvorrichtung (58, 62) am Jochelement (28),
- zwei Zugstreben (40, 42), die mit der Hubvorrichtung (62, 58, 52) parallel zueinander und voneinander beabstandet befestigt sind und Greifvorrichtungen (64, 68) an ihren freien Enden haben, die zum Ergreifen von an einem Wirbel angebrachten Haltemitteln (72, 74) ausgebildet sind.

2. Vorrichtung zum Einrichten von Wirbeln der menschlichen Wirbelsäule, mit
- einem länglichen jochartigen Element (80, 80a, 80b), das horizontal verläuft,
- vertikalen Beinen (74,78), die an den Enden des Jochelementes (80, 80a, 80b) befestigt sind und Füße (82, 86, 82a, 82b, 86a, 86b) an ihren unteren Enden haben, von denen einer (92, 82a, 82b) Mittel hat, die zum Befestigen am Steißbein eines Patienten mittels einer durch ein Loch (84a) im Fuß (82, 82a, 82b) laufenden Steißbeinschraube geeignet sind, und der andere (86, 86a, 86b) ringförmig ist, um auf der Schulter eines Patienten aufzuliegen,
- einem Schlitten (88), der verschieblich entlang des Jochelementes (80, 80a, 80b) aufgebaut ist,
- zwei Zugstreben (90), die am Schlitten (88) parallel zueinander und voneinander beabstandet befestigt und höhenverstellbar sind, wobei die Zugelemente (90) an ihren freien Enden eine Greifvorrichtung haben, die zum Ergreifen von an einem Wirbel befestigten Haltemitteln ausgebildet sind.

3. Vorrichtung nach Anspruch 1 oder 2, dadurch gekennzeichnet, daß die Haltemittel von einer Knochenfortsatz-Schraube oder einem durch ein Loch eines Wirbels gezogenen Draht gebildet werden und mit den Zugstreben durch einen daran befestigten Haken zusammenwirken.

4. Vorrichtung nach einem der Ansprüche 1 bis 2, dadurch gekennzeichnet, daß die Beine (16, 18) lösbar am Tisch befestigt sind.

5. Vorrichtung nach einem der Ansprüche 1 bis 4, dadurch gekennzeichnet, daß die Beine abnehmbar am Jochelement (28) befestigt sind.

6. Vorrichtung nach einem der Ansprüche 1 bis 5, dadurch gekennzeichnet, daß die Beine (16a) gegenüber dem Jochelement (28) bzw. dem Tisch drehbar sind.

7. Vorrichtung nach Anspruch 6, dadurch gekennzeichnet, daß das Jochelement (28a) gegenüber den Beinen um eine Längsachse oder eine parallel zur Längsachse verlaufenden Achse des Jochelementes drehbar ist.

8. Vorrichtung nach einem der Ansprüche 1 bis 7, dadurch gekennzeichnet, daß die Beine (16,18) gegenüber dem Jochelement mit Hilfe von Langlöchern (24,28) verschiebbar sind und in einer beliebigen Stellung fixiert werden können.

9. Vorrichtung nach einem der Ansprüche 1 bis 8, dadurch gekennzeichnet, daß die Zugstreben (40,42,90) längenverstellbar sind.

10. Vorrichtung nach Anspruch 8 oder 9, dadurch gekennzeichnet, daß die Zugstreben (40,42) an einer Hubplatte (52) befestigt sind, die mittels einer Spindel (58) gegenüber dem Jochelement (28) höhenverstellbar ist.

11. Vorrichtung nach einem der Ansprüche 1 bis 10, dadurch gekennzeichnet, daß die Zugstreben (40,42,90) aus zwei Teilen (44,48,46,50,92,94) bestehen, wobei ein Teil an der Hubvorrichtung befestigt ist und der zweite Teil am ersten Teil hängt.

12. Vorrichtung nach Anspruch 11, dadurch gekennzeichnet, daß die unteren Enden der Zugstreben gabelartige Haken zum Eingriff an den Köpfen (68,70) der Knochenfortsatz-Schrauben (72,74) von unten oder Haken zum Eingriff in einen schlaufenförmigen Haltedraht haben.

13. Vorrichtung nach einem der Ansprüche 2 bis 12, dadurch gekennzeichnet, daß mindestens ein Bein (74) gegenüber dem Jochelement (80) drehbar ist, jedoch in jeder Winkelstellung fixierbar ist.

## Revendications

1. Dispositif pour le repositionnement de vertèbres de la colonne vertébrale humaine, comportant
- un élément allongé (28) analogue à un joug s'étendant horizontalement,
- des pieds verticaux (16, 18) reliés aux extrémités de l'élément (28) analogue à un joug, lesdits pieds étant réalisés de façon à être supportés le long de côtés longitudinaux opposés d'une table qui est conçue pour supporter un patient étendu sur elle, lesdits pieds pouvant être déplacés par rapport à ladite table,
- des moyens d'élévation (58, 62) sur ledit élément (28) analogue à un joug,
- deux éléments de liaison (40, 42) reliés aux moyens d'élévation (62, 58, 52) dans une disposition espacée s'étendant en parallèle et ayant des moyens de prise (64, 66) à leurs extrémités libres, conçus pour engager des moyens de maintien (72, 74) reliés à une vertèbre.

2. Dispositif pour le repositionnement de vertèbres de la colonne vertébrale humaine, comportant
- un élément allongé (80, 80a, 80b) analogue à un joug s'étendant horizontalement,
- des pieds verticaux (74, 78) reliés aux extrémités de l'élément (80, 80a, 80b) analogue à un joug, lesdits pieds (74, 78) ayant des parties de base (82, 86, 82a, 82b, 86a, 86b) à leurs extrémités inférieures, l'une (92, 82a, 82b) desdites parties de base ayant des moyens conçus pour être reliés au sacrum d'un patient par l'intermédiaire d'une vis de sacrum passant dans un trou (84) de ladite partie de base (82, 82a, 82b) et les autres parties de base (86, 86a, 86b) étant en forme d'anneaux pour être supportées par la région des épaules d'un patient,
- un moyen coulissant (88) réalisé de façon à être déplacé le long dudit élément (80, 80a, 80b) analogue à un joug,
- deux éléments de liaison (90) reliés audit moyen coulissant (88) dans une disposition espacée, s'étendant en parallèle, et conçus pour pouvoir être réglés en hauteur, lesdits éléments de liaison (90) ayant des moyens de prise à leurs extrémités libres, conçus pour engager des moyens de maintien reliés à une vertèbre.

3. Dispositif selon la revendication 1 ou 2, dans lequel lesdits moyens de maintien sont définis par une vis pédiculaire ou un fil métallique tiré à travers le trou d'une vertèbre, lesdits moyens de maintien coopérant avec lesdits éléments de liaison au moyen d'un crochet qui leur est relié.

4. Dispositif selon l'une des revendications 1 à 3, dans lequel lesdits pieds (16, 18) peuvent être reliés de façon amovible à ladite tête.

5. Dispositif selon l'une des revendications 1 à 4, dans lequel lesdits pieds (16, 18) sont montés de façon amovible sur ledit élément (28) analogue à un joug.

6. Dispositif selon l'une des revendications 1 à 5, dans lequel lesdits pieds (16a) peuvent pivoter par rapport audit élément (28) analogue à un joug ou à ladite table, respectivement.

7. Dispositif selon la revendication 6, dans lequel ledit élément (28a) analogue à un joug peut pivoter par rapport auxdits pieds autour d'un axe longitudinal ou d'un axe parallèle à l'axe longitudinal dudit élément analogue à un joug.

8. Dispositif selon l'une des revendications 1 à 7, dans lequel lesdits pieds (16, 18) peuvent être déplacés par rapport audit élément analogue à un joug à travers des trous allongés (24, 26) et peuvent être attachés fixement dans n'importe quelle position.

9. Dispositif selon l'une des revendications 1 à 8, dans lequel lesdits éléments de liaison (40, 42, 90) sont réglables dans leur longueur.

10. Dispositif selon la revendication 8 ou 9, dans lequel lesdits éléments de liaison (40, 42) sont attachés à une plaque d'élévation (52) qui est réglable en hauteur au moyen d'une broche (58) par rapport audit élément (28) analogue à un joug.

11. Dispositif selon l'une des revendications 1 à 10, dans lequel les éléments éléments de liaison (40, 42, 90) sont constitués d'une paire de pièces (44, 48, 46, 50, 92, 94), une pièce étant reliée auxdits moyens d'élévation et ladite seconde pièce étant suspendue de façon flottante sur la première pièce.

12. Dispositif selon la revendication 11, dans lequel les extrémités inférieures desdits éléments de liaison ont des crochets analogues à des fourches pour engager les têtes (68, 70) de vis pédiculaires (72, 74) par le dessous ou pour porter les crochets destinés à réaliser un engagement avec un fil métallique de maintien en forme de boucle.

13. Dispositif selon l'une des revendications 2 à 12, dans lequel au moins un pied (74) peut pivoter par rapport audit élément (80) analogue à un joug, mais peut être fixé solidement dans n'importe quelle position angulaire.
